# EUROPEAN PATENT SPECIFICATION

(11) **EP 1 644 938 B1**
(45) Date of publication and mention of the grant of the patent: **16.11.2011**
(21) Application number: 04737456.6
(22) Date of filing: 24.06.2004
(51) Int. Cl.: G21F 1/10, G21F 3/035, A61B 19/04

(54) **RADIATION PROTECTION MATERIAL, ESPECIALLY FOR USE AS RADIATION PROTECTION GLOVES**
STRAHLUNGSSCHUTZMATERIAL INSBESONDERE ZUR VERWENDUNG ALS STRAHLUNGSSCHUTZHANDSCHUHE
MATERIAU DE RADIOPROTECTION, DESTINE A ETRE UTILISE NOTAMMENT EN TANT QUE GANTS DE RADIOPROTECTION

(30) Priority: 25.06.2003 US 603305
(43) Date of publication of application: 12.04.2006
(73) Proprietor: WRP Asia Pacific Sdn. Bhd., Selangor Darul Ehsan (MY)
(72) Inventor: THIESS, Axel,, 43900 Sepang (Selangor Darul Ehsan) (MY); WONG, Ah Kiew,, 43900 Sepang (Selangor Darul Ehsan) (MY); LEW, Kwong An,, 43900 Sepang (Selangor Darul Ehsan) (MY); Jayaraman, Sarala, Devi,, 43900 Sepang (Selangor Darul Ehsan) (MY)
(74) Representative: Wright, Howard Hugh Burnby
(86) International application number: PCT/AU2004/000834
(87) International publication number: WO 2004/114323

(56) References cited:
- WO-A1-02/073627
- WO-A2-03/016029
- JP-A- 3 012 598
- US-A- 4 938 233
- US-A- 5 001 354
- US-A- 6 048 379
- US-A1- 2003 168 637
- US-A1- 2003 168 637
- US-B1- 6 517 774
- US-B1- 6 608 129
- PATENT ABSTRACTS OF JAPAN & JP 2001 124892 A (SUMITOMO RUBBER IND LTD) 11 May 2001
- PATENT ABSTRACTS OF JAPAN & JP 03 012 598 A (ASUKU:KK ET AL.) 21 January 1991

## Description

### Technical Field of the Invention

The invention concerns a radiation protection material, especially for use as radiation protection gloves and processes for their manufacture

### Background of the Invention

Various medical procedures require physicians and other personnel to work in areas prone to electromagnetic radiation exposure, to include exposure to X-rays, gamma-rays, and other types of radiation. For example, during many diagnostic, detection and guidance procedures, surgeons and other medical staff may work in a field of operation that is irradiated with X-rays to allow for the use of a fluoroscopic viewing screen. These personnel are thus exposed to doses of radiation that may exceed acceptable safety levels or to long-term exposure of low dosage level radiation Radiation exposure, even to low levels of X-rays, is known to produce a number of detrimental side effects. Medical personnel who work with X-rays and X-ray equipment thus require protection from such radiation exposure with protective garments or gloves that limit or attenuate the amounts of radiation received.

Accordingly, radiation protection garments that shield specific areas of the body sensitive to such radiation exposure are well known in the art. Such garments typically include coats, aprons, gloves and various shields having radiation absorbent materials therein to attenuate the radiation. The materials used to make such garments have been made from polymer mixtures having radiation attenuating materials mixed therein. The radiation attenuating materials of prior art mixtures have comprised lead, lead oxide, or other lead salts. Such attenuating materials were used, for example, in U.S. Patent No. 3,185,751.

U.S. Patent No. 3,185,751, which issued to S. D. Sutton on May 25, 1965 ("the Sutton patent"), is for the manufacture of latices, dispersions and compounds of polymeric organic material containing metal. The radiation protection material of this patent, which is used to make radiation protection gloves, comprises a middle layer of natural rubber latex containing lead particles arranged therein to attenuate the radiation intensity of scattered X-rays. The layer is formed by dipping a shaped former into a solution of matrix material followed by vulcanisation of the formed material. This layer is then covered on both sides with additional layers of material not having lead particles therein.

Although the lead particles of the Sutton patent proved effective in attenuating radiation, it has been found that lead powder promotes vulcanisation of the matrix solution in the liquid state before it has hardened. Thus, if the latex mixture has a relatively high lead content, it cannot be used for the continuous production of radiation protection gloves, as the matrix solution containing lead particles rapidly deteriorates and becomes unusable. Furthermore, the processing of lead is fundamentally undesirable for health reasons due to the fact that lead compounds are toxic materials. This toxicity may impose additional costs on the manufacture and/or user of such materials due to the required compliance with regulations relating to their handling and disposal.

Thus, there is a need for an invention that avoids the foregoing disadvantages. This invention thus arises from the task of finding a radiation protection material that is lead-free and in which radiation absorbing particles can be extremely homogeneously distributed.

### Summary of the Invention

According to the present invention there is provided a radiation protection material for use in radiation protection gloves comprising: at least one layer of a polymeric material of rubber having distributed therein radiation absorbing particles and a viscosity increasing agent for increasing the viscosity of the polymeric material so as to reduce the speed of sedimentation of said radiation absorbing particles, the radiation absorbing particles attenuating the intensity of scattered radiation; and at least one layer of a polymer coating on an inner surface thereof that reduces a surface friction of the inner surface of the radiation protection material with respect to hands.

Preferably, the viscosity increasing agent is a cellulose derivative (other than said polymeric material of rubber).

Accordingly, there is provided a radiation protection material made of a polymeric material having radiation absorbing or attenuating particles and a viscosity increasing agent distributed therein. In one embodiment of the invention, the radiation protection material can be formed into radiation protection gloves and may comprise natural rubber latex radiation absorbing particles, and a cellulose derivative (other than the rubber latex), which by increasing the viscosity of the matrix material effectively reduces the speed of sedimentation of the radiation absorbing particles suspended therein, thereby enabling such gloves to be manufactured by a dipping process.

Commercially available prevulcanised natural rubber latex, commonly known as PV, is particularly suitable for manufacturing these radiation protection gloves as it is found to have exceptionally high latex stability and can accept a high loading of up to twice its weight of radiation protection material without the whole matrix material undergoing premature coagulation. The resulting gloves formed from this material also have adequate mechanical strength an physical properties. The radiation protection gloves, which are lead-free, comprise at least one layer of material, with multiple layers being successively formed. The radiation adsorbing particles distributed within the radiation protection material of the gloves can comprise particles of metallic tin, tin-oxide, antimony-tin oxide, bismuth oxide, tungsten oxide, or mixtures of the same. The minute particle size of these radiation absorbing particles enhance their homogeneous dispersal within the material because, as a given particle size becomes more fine, it has a slower rate of sedimentation within the matrix material.

The radiation protection material is shaped by dipping a form or pattern, e.g. a hand pattern, into coagulant and then into a solution of matrix material in a through-flow bath. Leaching, drying and preliminary finishing operations such as beading or trimming then follow. The latex products may be vulcanised in circulating hot air, steam, or hot water. Dipping, followed by vulcanisation can be repeated several times, with finishing operations to include washing and drying.

### Brief Description of the Drawings

In the drawings:
FIG. 1 is a top view of a radiation protection glove made of one embodiment of the radiation protective material; and
FIG. 2 is a sectional view showing at least one layer of the radiation protection material of the glove;
FIG. 3 is a flow chart of one embodiment of the process of making radiation protection gloves using the radiation protection material; and
FIG. 4 is a flow chart showing one embodiment of the post treatment process of the radiation protection gloves made of the radiation protection material.

### Description of the Preferred Embodiment

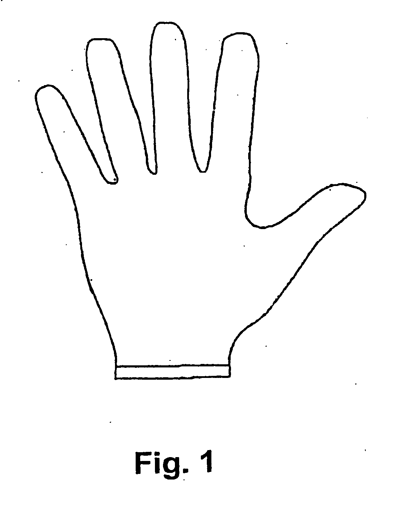

The invention provides for a radiation protection material made of at least one layer of a polymeric material having radiation absorbing or attenuating particles and a cellulose derivative distributed therein. The at least one layer of polymeric material may comprise about 20% to 40% by dry weight of rubber and about 60% to 80% by dry weight of radiation absorbing particles, preferably about 33% by dry weight of rubber and about 67% by dry weight of radiation absorbing particles. In one embodiment of the invention, the radiation protection material can be formed into radiation protection gloves, as shown in , FIG. 1. The polymeric material may be a rubber material made from polyisoprene rubber, (both natural and synthetic), polybutadiene rubber, styrene-butadiene rubber, nitrile rubber, butyl rubber, ethylene-propylene rubber, neoprene rubber, silicone rubber, polysulfide rubber, urethane rubber or other similar substances.

In the preferred embodiment of the invention, natural rubber latex, a type of polyisoprene rubber produced naturally from rubber trees, is used. The natural rubber latex may contain the usual compounding ingredients such as surfactants, vulcanising agents, activators, accelerators, antioxidants, pigments, antifoam agents and pH regulators in conventional amounts as needed to make gloves with the desired mechanical strength. Preference is given to the use of a commercially available ammoniac prevulcanised (PV) natural rubber latex where the preferred pH-value of this PV latex is greater than about 7.0, preferably about 10.0 to 11.0. The dry rubber content of this PV latex is about 50 to 70% by weight, preferably about 60% by weight, with an ammonia content between about 0.4 and 0.8% by weight, preferably about 0.6% by weight.

In addition to the radiation absorbing particles, the latex matrix mixture may also contain a cellulose derivative, such as methylcellulose (metholose) or some other water-soluble, cellulose ether. This additive, a water-soluble ether available in powder and granular forms, increases the viscosity of the matrix material mixture when dissolved and thus reduces the speed of sedimentation of the radiation absorbing particles therein. In this way, the heavy radiation absorbing particles can be held in suspension and distributed uniformly within the matrix material mixture. In one embodiment, the cellulose derivative within the at least one layer of polymeric material comprises about 0.1 to 0.4% by dry weight, preferably about 0.25% by dry weight.

In another embodiment of the mixture, natural rubber latex is used and 200 parts by dry weight of radiation absorbing particles are added to this latex containing 100 parts by dry weight of rubber (phr) and the usual compounding ingredients. The radiation absorbing particles are added to this latex compound in the form of a liquid dispersion, which in turn is prepared beforehand by ball-milling a typical composition as shown in Table 1.

| **TABLE 1** | | | |
|---|---|---|---|
| **RADIATION ABSORBING PARTICLE DISPERSION FORMULATION** | | | |
| **Material** | | **Pbw (dry)** | **Wet Weight (Kg)** |
| 100% | Radiation Absorbing Particle | 100.00 | 100.00 |
| 20% | Teric | 0.20 | 1.00 |
| 3% | Metholose | 0.10 | 3.33 |
| 3% | Latekoll D | 0.24 | 8.00 |
| | Deionized Water | - | 23.53 |
| | **Total** | **100.54** | **135.86** |

The typical properties of this dispersion as added to the latex compound are shown in Table 2.

| **TABLE 2** | |
|---|---|
| **TYPICAL RADIATION ABSORBING** | **PARTICLE DISPERSION PROPERTIES** |
| TSC% | 72.0 - 76.0 |
| pH | Min 9.0 |
| Viscosity (cps), Spindle 3 @ 30rpm | Min 900 |

This high mix proportion of the radiation absorbing particles to the latex compound is made possible by the addition of the methylcellulose, which in increasing the viscosity of the material mixture, reduces the sedimentation speed of the radiation absorbing particles to ensure a homogeneous distribution of the radiation absorbing particles within the mixture. In a preferred embodiment of the mixture, the composition of materials using prevulcanised natural rubber latex is as shown in Table 3.

| **TABLE 3** | | | |
|---|---|---|---|
| **RADIATION LATEX COMPOUND FORMULATION BASED ON PREVULCANISED NATURAL RUBBER LATEX** | | | |
| **Material** | | **Phr** | **Wet Weight (Kg)** |
| 60% | Prevulcanised Natural Rubber Latex (PV) | 100.00 | 166.67 |
| 20% | Emulvin W | 0.10 | 0.50 |
| 24% | Black Pigment | 0.012 | 0.05 |
| 74% | Radiation Absorbing particle | 200.00 | 270.27 |
| 100% | Coagulant WS | 0.20 | 0.20 |
| 3% | Metholose | 0.50 | 16.67 |
| | Delonized Water | - | 46.99 |
| | Total | 300.812 | 501.35 |

The physical properties of this material mixture, as used to produce gloves by a dipping process, are shown in Table 4.

| **TABLE 4** | |
|---|---|
| **TYPICAL RADIATION LATEX COMPOUND PROPERTIES** | |
| TSC% | 56.0 - 62.0 |
| pH | Min 9.0 |
| Viscosity (cps), Spindle 3 @ 30rpm | 300 - 500 |

We have found that commercial prevulcanised (PV) natural rubber latex has an unusually high latex stability which makes it possible to accept a high loading of up to twice its dry rubber weight of radiation absorbing particles without affecting the overall colloidal stability of the matrix material mixture, i.e. avoiding premature coagulation. In this way, a highly homogeneous distribution of the particles is achievable with high reproducibility over extended period of time, which makes it possible to mass produce such gloves by a continuous dipping process.

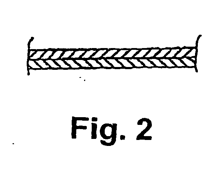

Turning to FIG. 2, the radiation protection material is lead-free and comprises at least one layer of material, with multiple layers being successively formed. The radiation protection glove can be made up of one, two or more than two layers. In the preferred embodiment of the invention, one layer is used if the final material thickness is about 0.3 mm or less. For material thicknesses of above 0.3 mm, two or more layers are used.

The radiation absorbing particles are all lead-free and can comprise bismuth alone or in combination with tungsten oxide, antimony-tin oxide and/or metallic tin. In one preferred embodiment, the radiation absorbing particles comprise about 60 to 90 % by weight of metallic tin powder and about 10 to 40% by weight of bismuth oxide particles. Alternatively, the radiation absorbing particles can comprise about 60 to 90 % by weight of tin oxide particles or antimony-tin oxide particles and about 10 to 40 % by weight of tungsten oxide particles.

According to another possibility, the radiation absorbing particles comprise about 40 to 60 % by weight of bismuth oxide particles and about 40 to 60 % by weight of tungsten oxide particles. In yet a further embodiment, the radiation absorbing particles can comprise about 40 to 60 % by weight of tin oxide particles or antimony-tin oxide particles, about 20 to 30 % by weight of tungsten oxide particles and about 20 to 30 % by weight of bismuth oxide particles.

Finally, the radiation absorbing particles can comprise about 60 to 90 % by weight of tin oxide particles or antimony-tin oxide particles and about 10 to 40 % by weight of bismuth oxide particles.

Other embodiments can utilize 100 % of a single composition of radiation absorbing particles instead of the above percentage combinations. According to these other embodiments, the radiation absorbing particles may be comprised entirely of bismuth oxide particles, tungsten oxide particles, tin oxide particles or antimony-tin oxide particles.

The particle size of all the radiation absorbing particles (tin, tin oxide, antimony-tin oxide, bismuth oxide, tungsten oxide) are less than about 10 µm, preferably less than about 6 µm. Such particles in the µm range are particularly suitable for homogeneous dispersal in the matrix compound material mixture. Because of their minute particle size, they exhibit an especially low sedimentation speed. Furthermore, radiation-absorbing particles with a particle size under about 2 µm, preferably under about 1 µm are preferred.

The radiation protection gloves can be made by dipping processes. These processes include simple straight dipping where one or more coats of the latex material mixture are applied with no coagulant being used and the coagulant dip process, where a form is first dipped into coagulant and then into the latex material mixture. Commonly used coagulants include calcium chloride, calcium nitrate, zinc nitrate, and acetic acid.

In the preferred embodiment using the coagulant dip process, the radiation protection material is shaped by dipping a form or pattern, e.g. a hand pattern, into coagulant and then into a latex compound of matrix material in a through-flow bath. The material is typically contained in a dipping tank provided with mechanical agitation and a temperature controlled jacket. The form, which is usually aluminium, porcelain, or stainless steel, may be dipped by manual control or automatic operation. It is essential to have uniformity in immersion and withdrawal rates. Care must be taken to avoid trapping air in the mixture, which causes pinholes and blister. After withdrawal of the form, the latex flow may be controlled in many ways, but is generally controlled by rotating the form to ensure an even distribution of the deposited latex.

Leaching, drying and preliminary finishing operations such as beading or trimming then follow. The latex products may be vulcanised in circulating hot air, steam, or hot water. If vulcanised with hot air, the vulcanisation process takes place in a through-flow oven. It is noted that vulcanisation may take place on or off the form. If cured on the form, dipping followed by vulcanisation can be repeated several times. The formed articles may be stripped wet or dry. Finishing operations include washing and drying.

In the preferred embodiment, a water dispersion of the submicron or micron-sized radiation absorbing particles and cellulose is first prepared by grinding the particles in water (about 75% concentration) in a ball-miller. This dispersion is then added slowly into a prevulcanised latex emulsion (containing the rubber) with constant stirring preferably at room temperature to yield a uniform liquid compound mixture as in normal latex compounding. The latex compound mixture essentially now contains a colloidal suspension of the submicron radiation absorbing particles being non-agglomerated and uniformly dispersed. A preferred radiation protection glove comprises two lead-free layers, as illustrated in FIG. 2, that are successively formed using a matrix material compound having the composition set forth in Table 3.

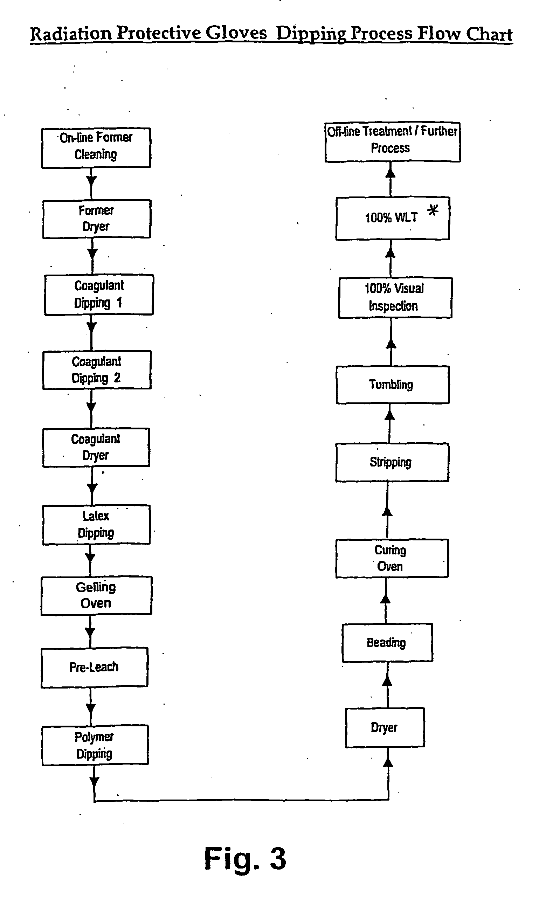

As illustrated in the flowchart of Figure 3, for the dipping of the base radiation protection gloves, the formers are first cleaned by dipping them successively into a solution of acid-based cleaner followed by an alkaline based former cleaning agent. The formers then further go through rotating mechanical brushes followed by rinsing with clean hot water at about 70 to 80°C to complete the cleaning. After cleaning, the formers are dried by circulating hot air in a former drying at a temperature of about 50 to 100°C for about 5 to 10 minutes. The formers are then dipped full length into coagulant 1 comprising about 30 to 40% calcium nitrate solution, which is then followed by dipping up to the wrist into a more dilute coagulant 2 comprising about 20 to 30 % calcium nitrate solution. This double coagulant dipping feature is to ensure that the final glove produced will have uniform thickness from cuff to fingertip.

After dipping the formers into the coagulant mixtures, they are dried in a coagulant dryer at a temperature of about 70 to 100 °C for about 5 to 10 minutes. Subsequent to drying, the formers are then dipped into the above described latex matrix compound which is stirred continuously but gently and maintained at a temperature of about 20 to 30 °C. The dwell time within the latex compound is about 5 seconds while the down and up times are about 8 seconds each. The coated formers are then moved to a gelling oven where they are exposed to hot air having a temperature of about 70 to 100 °C for a time duration of about 5 minutes.

After the mixture has gelled on the formers, the coated formers undergo a pre-leaching process by immersing them in over-flowing, clean, hot water at a temperature of about 60 to 80 °C for about 3 minutes. When pre-leaching is complete, the coated formers are then dipped momentarily into a polymer coating solution maintained at a temperature of about 20 to 30 °C. This polymer solution is a copolymer of acrylic acid and acrylic acid ester, and upon partial drying at a temperature of about 70 to 100 °C for about 3 minutes, will leave a thin polymer coating on the base glove. This polymer coating will be further bonded to the base glove upon subsequent curing of the whole glove to be described later. This polymer coating is very slick and has a lower surface friction with the human hands, which enables easy donning of the final formed glove without the need for any powder or other lubricating agents.

After partial drying, the polymer coated formers then undergo a beading process where the peripheral edges of the cuff openings of the dipped gloves are strengthened by rolling them into a solid bead (rubber band) of about 1 to 2 mm diameter. After the beading process is complete, the coated formers are then moved to a curing oven, which cures the beaded gloves by exposing them to recirculating hot air at a temperature of about 100 °C to 140 °C for about 60 to 100 minutes. When the gloves have cured, they are then manually stripped from the formers and further tumbled within a tumbler dryer at a temperature of about 70 to 90 °C for about 60 to 100 minutes to eliminate excessive powder and moisture from the gloves. This tumbling action also serves to complete curing of the gloves. The gloves thereafter undergo a 100 % visual inspection for visual defects. This is followed by 100 % water leak test (WLT) where the gloves are tested for pinholes/holes by filling each with about 1 litre of water and checking for leakages after about 2 minutes holding time.

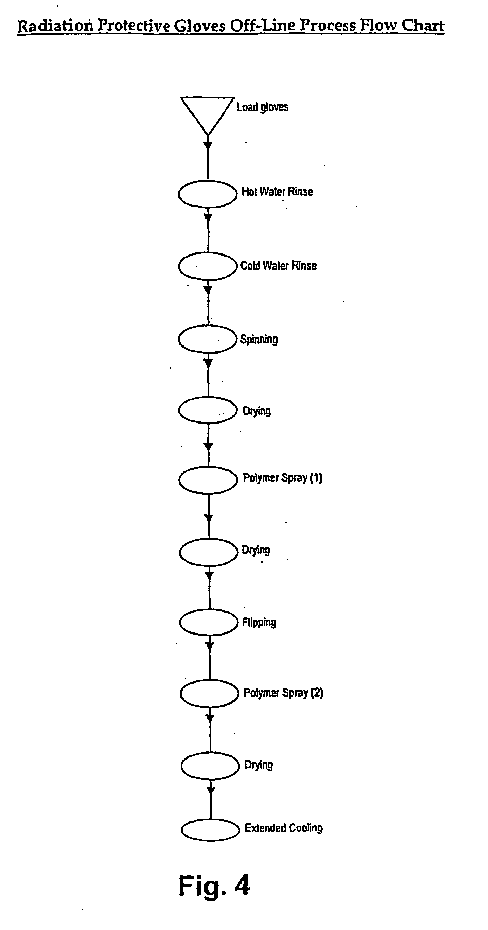

The formed gloves are further subjected to an off-line treatment/process as shown in Figure 4, the purpose of which is to: i) convert these gloves from powdered to powder free; ii) further enhance donnability especially with damp hands; iii) impart desired "skin-grip" surface finish on working side; and iv) prevent the gloves from sticking together on both the donning and working side (after cuffing) upon storage after sterilization. In the first stage of this off-line treatment, the formed gloves are first washed batch-wise with hot water at about 60 to 90 °C for at least about 10 minutes to remove powder and non-rubbers, including proteins that are inherently present in the natural rubber from the PV latex. The gloves are then washed with unheated cold water (ambient temperature about 30 °C) for about 10 minutes. They are then spun at about 400 RPM for about 10 minutes to extract surface water before being partially dried in tumbler dryers at a temperature of about 60 to 80 °C for about 30 minutes.

After this first partial drying of the gloves with their working side outside, at least one layer of a polymer (1), which is a polyacrylate polymer, is sprayed on to the outer surface of the gloves to get the desired surface (grip) finish, to reduce a surface drag on the outer surface and also to prevent stickiness of the working side upon storage after sterilization. The gloves are dried further at about 60 to 80 °C for about 10 minutes before they are removed from the tumblers and flipped (turned inside out) to get the donning side outside and the working side inside. They are now returned to another tumbler dryer and dried at about 60 to 80 °C for at least about 30 more minutes. During this second drying, at least one layer of a polymer (2), which comprises a copolymer of an acrylic acid and an acrylic acid ester or which comprises a cationic based super-surfactant, is sprayed in so as to coat the donning side uniformly to enhance the damp hand donnability of these gloves. After drying, the gloves are then cooled down to ambient temperature and then turned over (flipped) to get the correct configuration with the donning surface inside and the working surface outside. Finally, the gloves are pair-packed and sealed before being subjected to sterilization by gamma radiation like conventional surgical gloves.

The gloves, having radiation attenuating particles distributed therein, have the radiation attenuation characteristics, as measured according to DIN 6845/1 and IEC 1331-1 / ICRP 60/ICRU 51, as shown in Table 5. DIN-6815-1 is a German Standard for the "Testing of materials for radiation protection against x-rays and Gamma-rays," with DIN being an acronym for DEUTSCHE INDUSTRIE NORM (German Industrial Standard). IEC 1331-1 (International Electro-technical Commission) is a standard of attenuation properties of materials. ICRP 60 is the 60th recommendation of the International Commission of Radiation Protection, which is the governing body on all radiation issues. ICRU 51 is the 51st recommendation of the International Commission of Units.

| **TABLE 5** | | | | | | | | |
|---|---|---|---|---|---|---|---|---|
| **ATTENTUATION TEST RESULTS OF RADIATION PROTECTION GLOVES WITH THICKNESS 0.30mm** | | | | | | | | |
| **Material** | | | | | | **Attenuation Test Result** | | |
| **Samp le** | **ry rubber (% by weight)** | **Radiation Absorbing Particle (% by weight)** | | | | **60kV** | **80Kv** | **100kV** |
| | | **Bi₂O₃** | **WO₃** | **SnO** | **Sn** | | | |
| 1 | 33.0 | 16.7 | - | - | 50.3 | 49% | 43% | 36% |
| 2 | 33.0 | - | 16.7 | 50.3 | - | 41% | 29% | 23% |
| 3 | 33.0 | 67.0 | - | - | - | 58% | 49% | 41% |
| 4 | 33.0 | 33.5 | 33.5 | - | - | 54% | 40% | 34% |
| 5 | 33.0 | - | 67.0 | - | - | 41% | 33% | 24% |
| 6 | 33.0 | 16.7 | 16.7 | 33.5 | - | 54% | 42% | 34% |
| 7 | 33.0 | 33.5 | - | - | 33.5 | 56% | 47% | 40% |
| 8 | 33.0 | - | - | 67.0 | - | 29% | 26% | 23% |

The examples that follow describe some of these gloves which showed a maximum reduction in the radiation dose from secondary X-rays at 60 and 100 kV intensity of about 58 % (60 kV) and about 41 % (100kV) respectively at a glove thickness of 0.3 mm. The equivalent lead value lies between about 0.03 and 0.04 mm Pb.

Example 1: Glove comprising about 33% by dry weight natural rubber (NR) with about 16.7% bismuth oxide particles and about 50.3% metallic tin particles. At 0.3 mm glove thickness, attenuation at 60kV, 80kV and 100kV are 49 %, 43 % and 36 %, respectively.

Example 2: Glove comprising about 33% by dry weight natural rubber (NR) with about 16.7% tungsten oxide and about 50.3% tin oxide. At 0.3 mm glove thickness, attenuation at 60kV, 80kV and 100kV are 41 %, 29 % and 23 %, respectively.

Example 3: Glove comprising about 33% by dry weight natural rubber(NR) with about 67% bismuth oxide. At 0.3mm glove thickness, attenuation at 60kV, 80kV and 100kV are 58 %, 49 % and 41 %, respectively.

Example 4: Glove comprising about 33% by dry weight natural rubber (NR) with about 33.5% bismuth oxide about 33.5% tungsten oxide. At 0.3mm glove thickness, attenuation at 60kV, 80kV and 100kV are 54 %, 40 % and 34 %, respectively.

Example 5: Glove comprising about 33% by dry weight natural rubber (NR) with about 67% tungsten oxide. At 0.3mm glove thickness, attenuation at 60kV, 80kV and 100kV are 41 %, 33 % and 24 %, respectively.

Example 6: Glove comprising about 33% by dry weight natural rubber (NR) with about 16.7% bismuth oxide, about 16.7% tungsten oxide and about 33.5% tin oxide. At 0.3mm glove thickness, attenuation at 60kV, 80kV and 100kV are 54 %, 42 % and 34 %, respectively.

Example 7: Glove comprising about 33% by dry weight natural rubber (NR) with about 33.5% bismuth oxide and about 33.5% metallic tin. At 0.3mm glove thickness, attenuation at 60kV, 80kV and 100kV are 56 %, 47 % and 40 %, respectively.

Example 8: Glove comprising about 33% by dry weight natural rubber (NR) with about 67% tin oxide. At 0.3mm glove thickness, attenuation at 60kV, 80kV and 100kV are 29 %, 26 % and 23 %, respectively.

## Claims

1. A radiation protection material for use in radiation protection gloves comprising: at least, one layer of a polymeric material of rubber having distributed therein radiation absorbing particles and a cellulose derivative as a viscosity increasing agent for increasing the viscosity of the polymeric material so as to reduce the speed of sedimentation of said radiation absorbing particles, the radiation absorbing particles attenuating the intensity of scattered radiation; and at least one layer of a polymer coating on an inner surface thereof that reduces a surface friction of the inner surface of the radiation protection material with respect to hands.

2. The radiation protection material of claim 1 wherein the at least one layer of polymeric material comprises about 20 to 40% by dry weight of rubber and about 60 to 80% by dry weight of radiation absorbing particles.

3. The radiation protection material of claim 2 wherein the viscosity increasing agent is a cellulose derivative (other than said polymeric material of rubber).

4. The radiation protection of claim 3 wherein the at least one layer of polymeric material comprises about 33% by dry weight of rubber and about 67% by dry weight of radiation absorbing particles.

5. The radiation protection material of claim 4 wherein the cellulose derivative comprises about 0.1 to 0.4% by dry weight, preferably about 0.25% by dry weight.

6. The radiation protection material of claim 4 wherein the cellulose derivative comprises a water-soluble cellulose ether.

7. The radiation protection material of claim 6 wherein the water-soluble cellulose ether comprises methylcellulose.

8. The radiation protection material of claim 4 wherein the radiation absorbing particles comprise about 100% by weight of bismuth oxide particles.

9. The radiation protection material of claim 4 wherein the radiation absorbing particles comprise about 100% by weight of tungsten oxide particles.

10. The radiation protection material of claim 4 wherein the radiation absorbing particles comprise about 100% by weight of tin oxide particles.

11. The radiation protection material of claim 4 wherein the radiation absorbing particles comprise about 100% by weight of antimony-tin oxide particles.

12. The radiation protection material of claim 4 wherein the radiation absorbing particles comprise about 60 to 90% by weight metallic tin particles and about 10 to 40% by weight of bismuth oxide particles.

13. The radiation protection material of claim 4 wherein the radiation absorbing particles comprise about 60 to 90 % by weight of tin oxide particles and about 10 to 40 % by weight of tungsten oxide particles.

14. The radiation protection material of claim 4 wherein the radiation absorbing particles comprise about 60 to 90 % by weight of antimony-tin oxide particles and about 10 to 40 % by weight of tungsten oxide particles.

15. The radiation protection material of claim 4 wherein the radiation absorbing particles comprise about 40 to 60 % by weight of bismuth oxide particles and about 40 to 60 % by weight of tungsten oxide particles.

16. The radiation protection material of claim 4 wherein the radiation absorbing particles comprise about 40 to 60 % by weight of tin oxide, about 20 to 30 % by weight of tungsten oxide particles, and about 20 to 30 % by weight of bismuth oxide particles.

17. The radiation protection material of claim 4 wherein the radiation absorbing particles comprise about 40 to 60 % by weight of antimony-tin oxide particles, about 20 to 30 % by weight of tungsten oxide particles, and about 20 to 30 % by weight of bismuth oxide particles.

18. The radiation protection material of claim 4, wherein the radiation absorbing particles comprise about 60 to 90 % by weight of tin oxide particles and about 10 to 40 % by weight of bismuth oxide particles.

19. The radiation protection material of claim 4 wherein the radiation absorbing particles comprise about 60 to 90 % by weight of antimony-tin oxide particles and about 10 to 40 % of bismuth oxide particle.

20. The radiation material of claim 4 wherein the at least one layer of polymeric material is formed by dipping a pattern into the material and vulcanising the material on the pattern.

21. The radiation protection material of claim 1 wherein the polymeric material of rubber is selected from the group consisting of polyisoprene rubber, polybutadiene rubber, styrene-butadiene rubber, nitrile rubber, butyl rubber, ethylene-propylene rubber, neoprene rubber, silicone rubber, polysulfide rubber and urethane rubber.

22. The radiation protection material of claim 21 wherein the polyisoprene rubber is comprised of a natural rubber latex.

23. The radiation protection material of claim 22 wherein the natural rubber latex comprises about 60% by dry weight of rubber and about 0.4 to 0.8% by weight of ammonia prior to a vulcanisation of the material.

24. The radiation protection material of claim 22 wherein the natural rubber latex is a prevulcanised natural rubber latex having a pH-value in the range of about 10 to 11.

25. The radiation protection material of claim 4 wherein the at least one layer of polymeric material comprises at least two layers.

26. The radiation protection materials of claim 1 wherein the at least one layer of polymer coating comprises a copolymer of an acrylic acid and an acrylic acid ester.

27. The radiation protection material of claim 1 further comprising at least one layer of a cationic surfactant to improve the lubricity and donnability of the gloves with respect to damp hands.

28. The radiation protection material of claim 4 further comprising at least one layer of a polymer coating on an outer surface of the at least one layer of material that reduces a stickiness of the surface.

29. The radiation protection material of claim 28 wherein the at least one layer of polymer coating reduces a surface drag of the outer surface.

30. The radiation protection material of claim 28 wherein the at least one layer of polymer coating material comprises a polyacrylate.

31. The radiation protection material of claim 4 wherein the radiation adsorbing particles have a particle size of less than about 10 µm.

32. The radiation protection material of claim 3 wherein the radiation absorbing particles have a particle size of less than about 6 µm.

33. The radiation protection material of claim 3 wherein the radiation absorbing particles have a particle size of less than about 2 µm.

## Patentansprüche

1. Strahlenschutzmaterial zur Anwendung in Strahlenschutzhandschuhen, das aufweist: mindestens eine Schicht eines Polymerisat-Materials aus Gummi mit darin verteilten, Strahlung absorbierenden Teilchen und einem Cellulose-Derivat als einem die Viskosität steigendem Wirkstoff zum Erhöhen der Viskosität des Polymerisatmaterials, um die Absetzgeschwindigkeit der Strahlung absorbierenden Teilchen zu verringern, wobei die Strahlung absorbierenden Teilchen die Intensität von Streustrahlung absorbieren, und mindestens eine Lage einer Polymerisat-Beschichtung auf einer Innenfläche davon, die eine Oberflächenreibung der Innenfläche des Strahlenschutzmaterials im Verhältnis zu den Händen verringert.

2. Strahlenschutzmaterial nach Anspruch 1, bei dem die mindestens eine Schicht aus Polymerisat-Material etwa 20 % bis 40 % des Trockengewichts aus Gummi und etwa 60 % bis 80 % des Trockengewichts aus Strahlung absorbierenden Teilchen aufweist.

3. Strahlenschutzmaterial nach Anspruch 2, bei dem der die Viskosität steigernde Wirkstoff ein Cellulose-Derivat (abgesehen vom Polymerisat-Material aus Gummi) ist.

4. Strahlenschutzmaterial nach Anspruch 3, bei dem die mindestens eine Schicht aus Polymerisat-Material etwa 33 % des Trockengewichts aus Gummi und etwa 67 % des Trockengewichts aus Strahlung absorbierenden Teilchen aufweist.

5. Strahlenschutzmaterial nach Anspruch 4, bei dem das Cellulose-Derivat etwa 0,1 % bis 0,4 % des Trockengewichts und vorzugsweise etwa 0,25 % des Trockengewichts ausmacht.

6. Strahlenschutzmaterial nach Anspruch 4, bei dem das Cellulose-Derivat einen wasserlöslichen Celluloseäther aufweist.

7. Strahlenschutzmaterial nach Anspruch 6, bei dem der wasserlösliche Celluloseäther Methylcellulose aufweist.

8. Strahlenschutzmaterial nach Anspruch 4, bei dem die Strahlung absorbierenden Teilchen etwa 100 Gew.-% Wismuthoxid-Teilchen aufweisen.

9. Strahlenschutzmaterial nach Anspruch 4, bei dem die Strahlung absorbierenden Teilchen etwa 100 Gew.-% Wolframoxid-Teilchen aufweisen.

10. Strahlenschutzmaterial nach Anspruch 4, bei dem die Strahlung absorbierenden Teilchen etwa 100 Gew.-% Zinnoxid-Teilchen aufweisen.

11. Strahlenschutzmaterial nach Anspruch 4, bei dem die Strahlung absorbierenden Teilchen etwa 100 Gew.-% Antimonzinnoxid-Teilchen aufweisen.

12. Strahlenschutzmaterial nach Anspruch 4, bei dem die Strahlung absorbierenden Teilchen etwa 60 Gew.-% bis 90 Gew.-% metallische Zinn-Teilchen und etwa 10 Gew.-% bis 40 Gew.-% Wismuthoxid-Teilchen aufweisen.

13. Strahlenschutzmaterial nach Anspruch 4, bei dem die Strahlung absorbierenden Teilchen etwa 60 Gew.-% bis 90 Gew.-% Zinnoxid-Teilchen und etwa 10 Gew.-% bis 40 Gew.-% Wolframoxid-Teilchen aufweisen.

14. Strahlenschutzmaterial nach Anspruch 4, bei dem die Strahlung absorbierenden Teilchen etwa 60 Gew.-% bis 90 Gew.-% Antimonzinnoxid-Teilchen und etwa 10 Gew.-% bis 40 Gew.-% Wolframoxid-Teilchen aufweisen.

15. Strahlenschutzmaterial nach Anspruch 4, bei dem die Strahlung absorbierenden Teilchen etwa 40 Gew.-% bis 60 Gew.-% Wismuthoxid-Teilchen und etwa 40 Gew.-% bis 60 Gew.-% Wolframoxid-Teilchen aufweisen.

16. Strahlenschutzmaterial nach Anspruch 4, bei dem die Strahlung absorbierenden Teilchen etwa 40 Gew.-% bis 60 Gew.-% Zinnoxid-Teilchen, etwa 20 Gew.-% bis 30 Gew.-% Wolframoxid-Teilchen und etwa 20 Gew.-% bis 30 Gew.-% Wismuthoxid-Teilchen aufweisen.

17. Strahlenschutzmaterial nach Anspruch 4, bei dem die Strahlung absorbierenden Teilchen etwa 40 Gew.-% bis 60 Gew.-% Antimonzinnoxid-Teilchen, etwa 20 Gew.-% bis 30 Gew.-% Wolframoxid-Teilchen und etwa 20 Gew.-% bis 30 Gew.-% Wismuthoxid-Teilchen aufweisen.

18. Strahlenschutzmaterial nach Anspruch 4, bei dem die Strahlung absorbierenden Teilchen etwa 60 Gew.-% bis 90 Gew.-% Zinnoxid-Teilchen und etwa 10 Gew.-% bis 40 Gew.-% Wismuthoxid-Teilchen aufweisen.

19. Strahlenschutzmaterial nach Anspruch 4, bei dem die Strahlung absorbierenden Teilchen etwa 60 Gew.-% bis 90 Gew.-% Antimonzinnoxid-Teilchen und etwa 10 Gew.-% bis 40 Gew.-% Wismuthoxid-Teilchen aufweisen.

20. Strahlenschutzmaterial nach Anspruch 4, bei dem die mindestens eine Schicht Polymerisat-Material durch Eintauchen eines Musters in das Material und Aufvulkanisieren des Materials auf das Muster gebildet wird.

21. Strahlenschutzmaterial nach Anspruch 1, bei dem das Polymerisat-Material aus Gummi ausgewählt ist aus der aus Polyisopren-Gummi, Polybutadien-Gummi, Styrol-Butadien-Gummi, Nitril-Gummi, Butyl-Gummi, Ethylen-Propylen-Gummi, Neopren-Gummi, Silicon-Gummi, Polysulfid-Gummi und Urethan-Gummi bestehenden Gruppe.

22. Strahlenschutzmaterial nach Anspruch 21, bei dem der Polyisopren-Gummi aus einem Naturgummilatex besteht.

23. Strahlenschutzmaterial nach Anspruch 22, bei dem der Naturgummilatex vor dem Vulkanisieren des Materials etwa 60 % des Trockengewichts aus Gummi und etwa 0,4 % bis 0,8 % Gew.-% Ammoniak aufweist.

24. Strahlenschutzmaterial nach Anspruch22, bei dem der Naturgummilatex ein vorvulkanisierter Naturgummilatex mit einem pH-Wert im Bereich von etwa 10 bis 11 ist.

25. Strahlenschutzmaterial nach Anspruch 4, bei dem die mindestens eine Schicht aus Polymerisatmaterial mindestens zwei Lagen aufweist.

26. Strahlenschutzmaterial nach Anspruch 1, bei dem die mindestens eine Schicht einer Polymerisat-Beschichtung ein Copolymerisat aus einer Acrylsäure und einem Acrylsäureester aufweist.

27. Strahlenschutzmaterial nach Anspruch 1 weiterhin mit mindestens einer Schicht eines cationischen grenzflächenaktiven Stoffs, um die Lubrizität und Anlegbarkeit der Handschuhe für feuchte Hände zu verbessern.

28. Strahlenschutzmaterial nach Anspruch 4 weiterhin mit mindestens einer Lage einer Polymerisat-Beschichtung auf einer Außenfläche der mindestens eine Materialschicht, die eine Klebrigkeit der Oberfläche verringert.

29. Strahlenschutzmaterial nach Anspruch 28, bei dem die mindestens eine Lage einer Polymerisat-Beschichtung einen Oberflächen-Zugwiderstand der Außenfläche verringert.

30. Strahlenschutzmaterial nach Anspruch 28, bei dem die mindestens eine Lage aus Polymerisat-Beschichtungsmaterial ein Polyacrylat aufweist.

31. Strahlenschutzmaterial nach Anspruch 4, bei dem die Strahlung absorbierenden Teilchen eine Teilchengröße von weniger als etwa 10 µm aufweisen.

32. Strahlenschutzmaterial nach Anspruch 3, bei dem die Strahlung absorbierenden Teilchen eine Teilchengröße von weniger als etwa 6 µm aufweisen.

33. Strahlenschutzmaterial nach Anspruch 3, bei dem die Strahlung absorbierenden Teilchen eine Teilchengröße von weniger als etwa 2 µm aufweisen.

## Revendications

1. Matériau de radioprotection pour utilisation dans des gants de radioprotection comprenant : au moins une couche d'un matériau polymère en caoutchouc dans lequel sont distribuées des particules absorbant les radiations et un dérivé cellulosique servant d'agent augmentant la viscosité pour augmenter la viscosité du matériau polymère afin de réduire la vitesse de sédimentation desdites particules absorbant les radiations, les particules absorbant les radiations atténuant l'intensité des radiations diffusées ; et au moins une couche d'un revêtement polymère sur une surface intérieure de celui-ci qui réduit le frottement de surface de la surface intérieure du matériau de radioprotection vis-à-vis des mains.

2. Matériau de radioprotection selon la revendication 1, dans lequel l'au moins une couche de matériau polymère comprend environ 20 à 40 % en poids sec de caoutchouc et environ 60 à 80 % en poids sec de particules absorbant les radiations.

3. Matériau de radioprotection selon la revendication 2, dans lequel l'agent augmentant la viscosité est un dérivé cellulosique (autre que ledit matériau polymère en caoutchouc).

4. Matériau de radioprotection selon la revendication 3, dans lequel l'au moins une couche de matériau polymère comprend environ 33 % en poids sec de caoutchouc et environ 67 % en poids sec de particules absorbant les radiations.

5. Matériau de radioprotection selon la revendication 4, dans lequel le dérivé cellulosique représente environ 0,1 à 0,4 % en poids sec, de préférence environ 0,25 % en poids sec.

6. Matériau de radioprotection selon la revendication 4, dans lequel le dérivé cellulosique comprend un éther cellulosique soluble dans l'eau.

7. Matériau de radioprotection selon la revendication 6, dans lequel l'éther cellulosique soluble dans l'eau comprend de la méthylcellulose.

8. Matériau de radioprotection selon la revendication 4, dans lequel les particules absorbant les radiations comprennent environ 100 % en poids de particules d'oxyde de bismuth.

9. Matériau de radioprotection selon la revendication 4, dans lequel les particules absorbant les radiations comprennent environ 100 % en poids de particules d'oxyde de tungstène.

10. Matériau de radioprotection selon la revendication 4, dans lequel les particules absorbant les radiations comprennent environ 100 % en poids de particules d'oxyde d'étain.

11. Matériau de radioprotection selon la revendication 4, dans lequel les particules absorbant les radiations comprennent environ 100 % en poids de particules d'oxyde d'antimoine-étain.

12. Matériau de radioprotection selon la revendication 4, dans lequel les particules absorbant les radiations comprennent environ 60 à 90 % en poids de particules d'étain métallique et environ 10 à 40 % en poids de particules d'oxyde de bismuth.

13. Matériau de radioprotection selon la revendication 4, dans lequel les particules absorbant les radiations comprennent environ 60 à 90 % en poids de particules d'oxyde d'étain et environ 10 à 40 % en poids de particules d'oxyde de tungstène.

14. Matériau de radioprotection selon la revendication 4, dans lequel les particules absorbant les radiations comprennent environ 60 à 90 % en poids de particules d'oxyde d'antimoine-étain et environ 10 à 40 % en poids de particules d'oxyde de tungstène.

15. Matériau de radioprotection selon la revendication 4, dans lequel les particules absorbant les radiations comprennent environ 40 à 60 % en poids de particules d'oxyde de bismuth et environ 40 à 60 % en poids de particules d'oxyde de tungstène.

16. Matériau de radioprotection selon la revendication 4, dans lequel les particules absorbant les radiations comprennent environ 40 à 60 % en poids d'oxyde d'étain, environ 20 à 30 % en poids de particules d'oxyde de tungstène, et environ 20 à 30 % en poids de particules d'oxyde de bismuth.

17. Matériau de radioprotection selon la revendication 4, dans lequel les particules absorbant les radiations comprennent environ 40 à 60 % en poids de particules d'oxyde d'antimoine-étain, environ 20 à 30 % en poids de particules d'oxyde de tungstène, et environ 20 à 30 % en poids de particules d'oxyde de bismuth.

18. Matériau de radioprotection selon la revendication 4, dans lequel les particules absorbant les radiations comprennent environ 60 à 90 % en poids de particules d'oxyde d'étain et environ 10 à 40 % en poids de particules d'oxyde de bismuth.

19. Matériau de radioprotection selon la revendication 4, dans lequel les particules absorbant les radiations comprennent environ 60 à 90 % en poids de particules d'oxyde d'antimoine-étain et environ 10 à 40 % de particules d'oxyde de bismuth.

20. Matériau de radioprotection selon la revendication 4, dans lequel l'au moins une couche de matériau polymère est formée par immersion d'un motif dans le matériau et vulcanisation du matériau sur le motif.

21. Matériau de radioprotection selon la revendication 1, dans lequel le matériau polymère en caoutchouc est choisi dans l'ensemble constitué par le caoutchouc de polyisoprène, le caoutchouc de polybutadiène, le caoutchouc de styrène-butadiène, le caoutchouc de nitrile, le caoutchouc de butyle, le caoutchouc d'éthylène-propylène, le caoutchouc de néoprène, le caoutchouc de silicone, le caoutchouc de polysulfure et le caoutchouc d'uréthane.

22. Matériau de radioprotection selon la revendication 21, dans lequel le caoutchouc de polyisoprène est constitué d'un latex de caoutchouc naturel.

23. Matériau de radioprotection selon la revendication 22, dans lequel le latex de caoutchouc naturel comprend environ 60 % en poids sec de caoutchouc et environ 0,4 à 0,8 % en poids d'ammoniac avant vulcanisation du matériau.

24. Matériau de radioprotection selon la revendication 22, dans lequel le latex de caoutchouc naturel est un latex de caoutchouc naturel prévulcanisé ayant une valeur de pH située dans la plage allant d'environ 10 à 11.

25. Matériau de radioprotection selon la revendication 4, dans lequel l'au moins une couche de matériau polymère comprend au moins deux couches.

26. Matériau de radioprotection selon la revendication 1, dans lequel l'au moins une couche de revêtement polymère comprend un copolymère d'un acide acrylique et d'un ester d'acide acrylique.

27. Matériau de radioprotection selon la revendication 1, comprenant en outre au moins une couche d'un tensioactif cationique pour améliorer le pouvoir lubrifiant et la facilité d'enfilage des gants dans le cas de mains humides.

28. Matériau de radioprotection selon la revendication 4, comprenant en outre au moins une couche d'un revêtement polymère sur une surface extérieure de l'au moins une couche de matériau qui réduit le collant de la surface.

29. Matériau de radioprotection selon la revendication 28, dans lequel l'au moins une couche de revêtement polymère réduit la résistance de frottement de la surface extérieure.

30. Matériau de radioprotection selon la revendication 28, dans lequel l'au moins une couche de matériau de revêtement polymère comprend un polyacrylate.

31. Matériau de radioprotection selon la revendication 4, dans lequel les particules absorbant les radiations ont une granulométrie inférieure à environ 10 µm.

32. Matériau de radioprotection selon la revendication 3, dans lequel les particules absorbant les radiations ont une granulométrie inférieure à environ 6 µm.

33. Matériau de radioprotection selon la revendication 3, dans lequel les particules absorbant les radiations ont une granulométrie inférieure à environ 2 µm.
